# EUROPEAN PATENT APPLICATION

(11) **EP 3 770 607 A1**
(43) Date of publication of application: **27.01.2021**
(21) Application number: 19777782.4
(22) Date of filing: 18.03.2019
(51) Int. Cl.: G01N 35/00, B07C 5/34, B07C 5/342

(54) **SCREENING METHOD FOR BIOLOGICAL SPECIMENS AND SCREENING SEPARATOR**

(30) Priority: 27.03.2018 KR 20180034942
(71) Applicant: Seoul National University R & DB Foundation, Seoul 08826 (KR)
(72) Inventor: KWON, Sung Hoon, Seoul 06294 (KR); LEE, Dae Won, Seoul 08783 (KR); LEE, Yong Ju, Goyang-si Gyeonggi-do 10423 (KR); KIM, Ok Ju, Seoul 02632 (KR); LEE, Choong Won, Seoul 08801 (KR)
(74) Representative: Prüfer & Partner mbB Patentanwälte · Rechtsanwälte
(86) International application number: PCT/KR2019/003115
(87) International publication number: WO 2019/190100

(57) **Abstract**

The present invention relates to a method and system for screening and separating biological specimens. The screening and separating method and system provide reliable results based on information related to the types, shapes, and positions of labeled products provided by an agent for accurate screening. In addition, the information related to the types, shapes, and positions can be combined to determine objective indices for the state of a target specimen, enabling rapid selection of the target specimen. The method and system for screening and separating biological specimens according to the present invention use an agent optimized to accurately and effectively create information on the selection of a target specimen and process images based on information on images stored in a server, enabling sensitive and highly reproducible screening evaluation.

## Description

### Technical Field

The present invention relates to a method and system for screening and separating biological specimens, and more specifically to a method for selectively isolating target biological specimens from a sample and a screening and separating system used in the method.

### Background Art

Mixtures of various types of specimens are present in environmental samples derived from natural environments and human blood samples. For example, a large number of microbial species are mixed in an environmental sample and blood cells consisting of erythrocytes, leukocytes, and thrombocytes are essentially present in human blood. In a special case, circulating tumor cells, bacteria, protozoa (for example, malaria) or erythrocytes infected with bacteria or viruses may be present in human blood. Technology for targeting and isolating specific cells are essential to pathologically analyze and understand the state of the specimens.

A molecular genetic method is used to determine whether target cells are present in a sample containing a mixture of impurities such as by-products. This method is carried out by constructing primers amplifying a gene present only in a specific material and performing polymerase chain reaction (PCR) to determine the presence or absence of the specific material. Another method for determining the presence of particular cells is carried out by spreading a sample containing a mixture of impurities such as by-products on slide glass, selectively staining the particular cells, and observing the stained particular cells with a microscope. However, both the molecular genetic method and the spreading-based method are carried out after cell death and require the use of expensive PCR equipment and high-magnification microscopes.

An immunofluorescence method based on the use of a fluorescent material that specifically adheres to the surface of a particular material is also known. In immunocytochemistry, this method is carried out by attaching a first antibody to an epitope of a specific protein, attaching an additional antibody to the first antibody, and observing a fluorescent molecule attached to the terminus of the additional antibody under a microscope to determine whether the specific protein is fluorescently stained in cell. When several proteins adhere to each other, immunofluorescence staining is performed using different antibodies and fluorescent colors for these proteins to determine sites where the proteins adhere to each other.

Although identified biological specimens are isolated in a non-contact mode rather than in a contact mode that is liable to damage the biological specimens, when the target materials are mixed with various foreign substances, target materials are difficult to isolate with high throughput in an automated manner. Further, there exist critical limitations in isolating intact biological specimens are still difficult. Because a sample including the biological specimens still requires various physical/chemical treatments.

In this connection, U.S. Patent Publication No. 2015-0322485 discloses a method of isolating biochemical molecules on a microarray substrate. The method includes applying energy in a contact or non-contact manner to isolate a desired cluster from a microarray substrate. As discussed above, however, it was found that when mixed with various foreign substances, target materials are difficult to isolate with high throughput in an automated manner.

### Detailed Description of the Invention

### Problems to be Solved by the Invention

Thus, the present inventors intended to provide a technology for screening and separating biological specimens by preparing labeled products of biological specimens, reading the types, shapes, and positions of the labeled products to objectively and rapidly select targets, and isolating targets in an automated manner with high efficiency despite the presence of foreign substances and by-products mixed with the targets.

That is, the present invention intends to provide a method for isolating targets from biological specimens by labeling biological specimens with a suitable agent, reading the types, shapes, and positions of the labeled products to provide information on the selection of targets, and objectively evaluating the information to isolate the targets in an automated manner, and a screening and separating system used in the method.

### Means for Solving the Problems

According to one aspect of the present invention, there is provided a method for screening and separating biological specimens, including: adding an agent to a sample including biological specimens to label the biological specimens and obtain a sample including labeled products; reading the types, shapes, and positions of the labeled products in the sample to select at least one target specimen among the labeled products; and isolating the selected target specimen or the unselected specimens.

According to a further aspect of the present invention, there is provided a method for screening and separating biological specimens, including: adding an agent to a sample including biological specimens to label the biological specimens and obtain a sample including labeled products; immobilizing the sample onto a substrate and reading the types, shapes, and positions of the labeled products to select at least one target specimen among the labeled products; and isolating the selected target specimen or the unselected specimens.

According to another aspect of the present invention, there is provided a system for screening and separating biological specimens, including: a stage mounted with a substrate on which a sample including labeled products is arranged; an image processing unit adapted to observe the labeled products and select at least one target specimen; and a laser platform adapted to isolate the target selected in the image processing unit.

### Effects of the Invention

The method and system for screening and separating biological specimens according to the present invention provide reliable results based on information related to the types, shapes, and positions of labeled products provided by an agent for accurate screening.

In addition, the information related to the types, shapes, and positions can be combined to determine objective indices for the state of a target specimen, enabling rapid selection of the target specimen.

The method and system for screening and separating biological specimens use an agent optimized to accurately and effectively create information on the selection of a target specimen and process images based on information on images stored in a server, enabling sensitive and highly reproducible screening evaluation.

### Brief Description of the Drawings

Fig. 1 is an exemplary flowchart illustrating a method for screening and separating biological specimens.
Fig. 2 is an exemplary flowchart illustrating a method for combining and sorting information related to types, shapes, and positions for screening and separating biological specimens.
Figs. 3 to 5 are exemplary diagrams illustrating the individual steps in the flowchart of Fig.
Fig. 6 is a schematic diagram illustrating the use of a laser platform for automatic isolation of the target specimen selected in Fig. 5.
Fig. 7 illustrates one embodiment of a system for screening and separating biological specimens.
Fig. 8 is an exemplary diagram of the laser platform used in Fig. 7.
Fig. 9 is a specific embodiment of Fig. 1.
Fig. 10 shows schematic diagrams comparing the selection of specimens based on only position information and adherend information without considering the shapes of the adherends in Comparative Example 1 with the selection of specimens based on further consideration of the shapes of adherends in Example 1.
Fig. 11 shows a high magnification image and fluorescence images of the specimens.

### Mode for Carrying out the Invention

As the technology described herein allows for various changes and numerous embodiments, particular embodiments will be illustrated in drawings and described in detail in the written description. However, this is not intended to limit the present invention to particular modes of practice, and it is to be appreciated that all changes, equivalents, and substitutes that do not depart from the spirit and technical scope of the technology described herein are encompassed in the technology described herein. Prior to the detailed description of the drawings, it is noted that in the specification, components are merely distinguished depending on their major functions. That is, two or more components described below may be combined into one or one component may be subdivided into two or more with different functions. In addition, it is to be understood that each of the components described below may additionally perform some or all of the functions of other components in addition to its major functions and some of the major functions of each of the components described below can also be performed by other components.

In implementing a method or operation, the respective steps of the method or operation may be carried out in a different order from that which is explicitly described unless the context clearly indicates otherwise. In other words, the respective steps may be carried out in the same order as described, substantially simultaneously, or in a reverse order.

The technology described herein relates to a method for screening and separating biological specimens. Specifically, the method includes: adding an agent to a sample (for example, a liquid sample) including biological specimens to label the biological specimens and obtain a sample including labeled products; reading the types, shapes, and positions of the labeled products in the sample to select at least one target specimen among the labeled products; and isolating the selected target specimen or the unselected specimens.

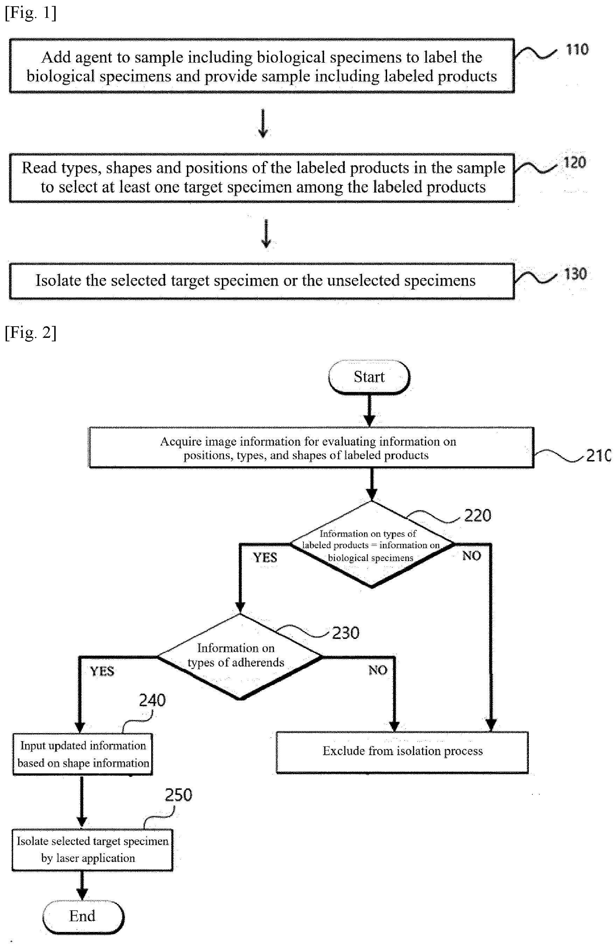

Fig. 1 is an exemplary flowchart illustrating the method for screening and separating biological specimens. In step 110 of Fig. 1, an agent is added to a sample (for example, a liquid sample) including biological specimens to label the biological specimens and obtain a sample including labeled products.

The sample may be derived from a living organism such as a human, an animal or a plant or from a natural environment such as sea water, river water, wastewater or soil. The sample derived from a living organism may include blood, saliva, pus, urine, feces, secretions, biopsy specimens, etc.

The sample may include both prokaryotic and eukaryotic cells. The sample may include mammalian cells, tumor cells, blood cells, prokaryotes (bacteria including cyanobacteria and archaea), filamentous fungi, yeasts, myxomycetes, basidiomycetes, unicellular algae, and protozoa. The sample may include a pathogenic microorganism. Examples of such pathogenic microorganisms include: viruses; rickettsia; bacteria, including cocci, bacilli, spirilla, and actinomycetes; fungi; and protozoa. In one example, the biological specimens may be selected from tissues, cells, nucleic acids, proteins, exosomes, metabolites, and mixtures thereof.

An agent is added to the sample including biological specimens to label the biological specimens and obtain a sample including labeled products.

The agent is used to label the biological specimens and may be, for example, an affinity material for labeling the biological specimens, a location information tracker or a combination thereof. The affinity material for the biological specimens may be a material that has characteristics such as chemical interaction, physical interaction or antigen-antibody reaction. Such affinity materials include nucleic acids, proteins, and combinations thereof, but are not limited thereto. For example, the affinity material may be a material that is biotinylated on the biological specimens. When biotinylated, the biological specimens form their specific complexes, for example, biotinylated complexes with streptavidin. Other examples of suitable affinity materials include: antibodies that target and have an affinity for antigens expressed on the surface of specific biological specimens; fluorescent molecules; luminescent molecules; and RNA- and DNA-based aptamers. Specific examples of affinity materials having an affinity for the biological specimens include beads, fluorescent molecules, luminescent molecules, antibodies, and aptamers. The affinity material is 1.0 µm or less or 0.01 nm to 1.0 µm in size, which is suitable for use as the agent.

Such binding enables the formation of complexes with the biological specimens. The binding can be performed in a variety of ways. For example, when the agent includes a material that specifically binds to the biological specimens simply by mixing the agent with the sample (for example, liquid sample) and gently shaking the mixture, sufficient labeling can be achieved. That is, when antibodies as the affinity material are applied to antigens as the biological specimens or beads are allowed to react with antibodies, a determination can be made whether desired biological specimens are present as adherends in information on the adherends to the biological specimens based on information on observed images of the labeled products.

Preferably, the provision of the location information tracker not only enables objective selection of the labeled products while facilitating the provision of information on the positions of the labeled products, but also is advantageous in that even subsequent isolation can be automated based on the position information.

The location information tracker is not limited as long as it can provide a color image without affecting the biological specimens. For example, the location information tracker may be selected from beads, fluorescent molecules, luminescent molecules, quantum dots, antibodies, aptamers, enzymes, DNAs, RNAs, PNAs, and combinations thereof.

Examples of suitable materials for the beads include silicon, magnetic materials, nanomaterials, polymers, and metals. The fluorescent molecules may be fluorescent dyes such as Alexa, Hoesct, cy3, cy5, bioluminescent materials, and auto-fluorescent materials, The luminescent molecules may be, for example, those of LED and luminescent materials. The quantum dots may be core-type quantum dots, core-shell quantum dots, and alloyed quantum dots. Specifically, the quantum dots may be core-shell quantum dots composed of elements in Groups II-V of the Periodic Table. The antibodies may be monoclonal antibodies, polyclonal antibodies, scFv antibodies, nanobodies, Fab antibodies, and chimeric antibodies. The enzymes may be HRP enzymes. The aptamers may be DNA- and RNA-based ones.

In step 120 of Fig. 1, the types, shapes, and positions of the labeled products are read to select at least one target specimen among the labeled products. That is, the technology disclosed in the present invention can offer an advantage in that the biological specimens can be screened without the need to immobilize the sample onto a substrate. Alternatively, the types, shapes, and positions of the labeled products may be read in a state in which the sample (for example, liquid sample) is immobilized onto a substrate, to select at least one target specimen among the labeled products. The substrate is not necessarily planar but is not limited as long as it can provide a surface for supporting the sample (for example, liquid sample). Examples of suitable substrates include, but are not limited to, slide glass, microbeads, nanoparticles, nanostructures, capillaries, microfluidic supports, porous structures, spongy structures, and dendrimers. The substrate may be made of glass, silicon or a polymeric material. For example, the substrate may be a microarray substrate or a next generation sequencing substrate integrated with biological specimens such as DNAs and proteins. The substrate includes a sacrificial layer for the purpose of reducing or eliminating damage to the biological specimens considering that a laser is applied in a subsequent process, but is not limited to this structure.

The sacrificial layer may be made of a light-transmitting metal oxide or a light-transmitting plastic material. For example, the sacrificial layer may be made of glass or silicon whose transmittance is reduced or whose absorbance is increased to increase energy absorption. Alternatively, the sacrificial layer may be coated on the surface of a solid such as glass or silicon. Alternatively, the sacrificial layer may be present in a solid such as glass or silicon. However, the location of the sacrificial layer is not particularly limited. The sacrificial layer is preferably made of a material free of optical distortion such that it is easy to determine whether laser light is accurately applied to the target specimen. When the target specimen is a biological material such as cells, the energy may be applied by an infrared laser that causes no damage to the target specimen. In this case, it is preferred that the sacrificial layer is evaporated by an infrared laser and transmits visible light to avoid interference with image observation of the biological material. The sacrificial layer is preferably formed using a metal oxide. The metal oxide may be, for example, indium tin oxide (ITO), indium zinc oxide (IZO), zinc oxide (ZnO), indium zinc tin oxide (IZTO), cadmium tin oxide (CTO) or indium gallium zinc oxide (IGZO) but is not limited thereto.

The step of selecting at least one target among the labeled products can be carried out by direct visual observation through optical microscopy or electron microscopy or by automated image processing to acquire information on the types, shapes, and positions of the labeled products.

The step of reading the types, shapes, and positions of the labeled products is preferably carried out through a combination of information on observed images, information on signals, including fluorescence signals, and information on coordinates.

The step of reading the types, shapes, and positions of the labeled products provides updated information by accumulating information on signals, including fluorescence signals, and information on observed images on information on coordinates. The information updating can be performed through a selection system including a database and a server but is not limited thereto. The database can store information on coordinates of the labeled products, information on signals (including fluorescence signals) of the labeled products, information on observed images of the labeled products, and information on constituent materials of the labeled products. The server accesses the information stored in the database to use information on constituent materials of the labeled products so that the biological specimens can be screened based on information on the types of adherends to the labeled products and information on the adhesion morphologies of the adherends provided from the information on coordinates of the labeled products, the information on signals of the labeled products, and the information on observed images of the labeled products.

Fig. 2 is a flowchart illustrating the operation of the screening system. Specifically, the flowchart of Fig. 2 illustrates a method for sorting the biological specimens and determining the screening order of the biological specimens based on a combination of information related to type, shape, and position for screening the biological specimens. Alternatively, any object recognition algorithm that can recognize the shapes, types, and positions of the labeled products and can be used in conjunction with an operable arithmetic unit may be used without limitation.

Referring to Fig. 2, first, when the information on the type of an adherend provided from information on signals (such as fluorescence signals) of the labeled products does not match information on the biological specimens as constituent materials of the labeled products, the server does not select the biological specimens.

When information on the type of an adherend provided from information on signals (such as fluorescence signals) of the labeled products matches information on the biological specimens as constituent materials of the labeled products, the server evaluates the information on the adhesion morphology of the adherend.

When information on the adhesion morphology of the adherend is the regular morphology, the server selects the corresponding biological specimen. The information on the selected target specimen is updated on information on observed images of the labeled products and the corresponding updated information is included in the database.

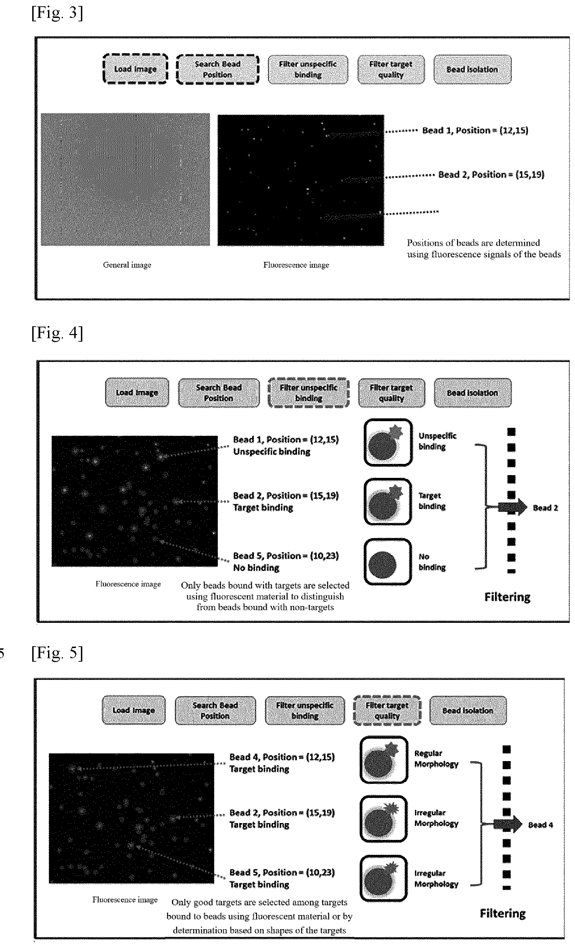

Figs. 3 to 5 are exemplary diagrams illustrating the individual steps in the flowchart of Fig. 2. Referring to Fig. 3 to 5, the method for screening and separating biological specimens according to the technology disclosed in the present invention can be carried out in the following order. Referring first to Fig. 3, a slide glass substrate on which cells as biological specimens, a specific affinity material, a location information tracker or a combination thereof are arranged is observed to determine the information on the position of the agent for labeling the biological specimens in the labeled products depending on the type of the location information tracker. The specific affinity material is selected from beads, fluorescent molecules, antibodies, aptamers, and combinations thereof, and the location information tracker is are selected from beads, fluorescent molecules, quantum dots, antibodies, aptamers, enzymes, DNAs, RNAs, PNAs, and combinations thereof. Referring to Fig. 4, the orange fluorescently labeled specimens (cells) in the image where the position information is determined are selected, and the green fluorescently labeled specimens and non-fluorescent specimens in the image are not selected. The green fluorescence indicates the binding of foreign matter or by-products. The screening information is preferably updated by accumulation on the position information of Fig. 3. Referring to Fig. 5, information on the adhesion morphologies of the adherends are confirmed in the image where information on the positions of the labeled products and information on the adherends are confirmed and only cells having the regular morphology are selected. As used herein, the term "regular morphology" refers to the inherent shape of biological specimens existing in nature or in engineered samples, unless otherwise mentioned. The regular morphology can be determined from the morphology of the fluorescent material or the morphology of the biological specimen bound to the extractant, including beads.

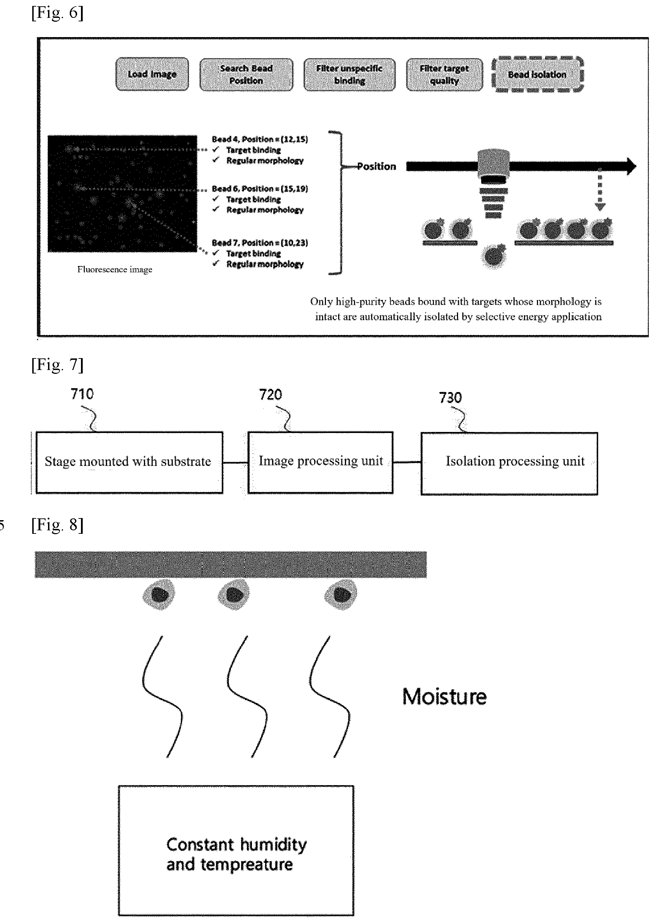

Then, in step 130 of Fig. 1, the selected target specimen or the unselected specimens are isolated, if needed. Preferably, the target specimen is isolated, for example, by applying a laser, preferably, in a non-contact mode or, if needed, in a contact mode in an order of the position information determined using the agent having a morphology close to the regular morphology, based on the position information inputted in a predetermined order, for example, in an order close to the regular morphology identified through a screening evaluation system or, if needed, in a reverse order thereof. Fig. 6 is a schematic diagram illustrating the use of a laser platform for automatic isolation of the target specimen selected in Fig. 5. Referring to Fig. 6 the target specimen is automatically isolated by selectively applying energy using an isolation system based on the updated information identified to have the regular morphology. That is, the target specimen can be retrieved in an automated manner by applying one or more isolation means selected from the group consisting of ultrasonic wave, radiation pressure, laser, pulling, and absorption to the target specimen based on the above-described updated information.

The method for screening and separating biological specimens disclosed in the present invention enables the selection of a desired target specimen from the biological specimens by observation through a suitable process such as imaging and easy isolation of the target specimen without contamination with the biological specimens and damage to the target specimen. In particular, the method disclosed in the present invention enables isolation of a high-purity biological specimen in an automated process regardless of whether foreign matter and by-products are present or absent in the sample, advantageously facilitating high-throughput screening and separating.

The method for screening and separating biological specimens provided in the present invention can be used in various applications. When a particular microorganism is identified in a sample that can be taken from the environment, the degree of contamination of the sample can be determined. Identification for the presence of specific bacteria in the blood is helpful in rapidly determining the presence of the bacteria in the blood from patients for quick prescription. Rapid and sensitive identification of an extremely small number of blood circulating tumor cells in the blood enables monitoring of the prognosis of patients undergoing cancer therapy or determination of the degree of metastasis. In addition, treatment with anticancer drugs, antibiotics, etc. and observation of the results enable a quick search for suitable therapeutic agents. Furthermore, cells with specific genes can be identified so that cancer can be diagnosed and mutation can be detected in a short time. That is, selective antigen and antibody screening of proteins as biological specimens is applicable to bioscience and biotechnology. Screening of specific cells as biological specimens can be applied to the medical field. Particularly, the screening and separating method described in the present invention is effectively applicable even when biological specimens are present in very small amounts compared to by-products (impurities).

According to another aspect of the technology disclosed in the present invention, there is provided a system for screening and separating biological specimens. Fig. 7 illustrates one embodiment of the system for screening and separating biological specimens. Referring to Fig. 7, the system 700 selectively isolates at least one target specimen among biological specimens and includes a stage 710 mounted with a substrate on which a sample including labeled products is arranged; an image processing unit 720 adapted to observe the labeled products and select at least one target specimen; and an isolation processing unit 730 adapted to isolate the target selected in the image processing unit 720. The stage 710 may include a mount adapted to mount a substrate and is preferably an electric stage for precise manipulation. The image processing unit 720 may include an imaging device for reading the type, position, and shape of a target specimen on a substrate, and specific examples thereof include an optical microscope or an electron microscope for observation.

In connection with the image processing unit 720, a computing device is explained to evaluate information on position, type, and shape based on information on images of labeled products, for convenience of description. The computing device is meant to include smart devices, PCs, and servers. For convenience of description, the computing device is assumed to determine information on position, type, and shape as either 1) satisfaction of information on position and type or 2) satisfaction of information on position, type, and shape.

The computing device should first acquire image information to evaluate information on the positions, types, and shapes of the labeled products (see 210 of Fig. 2). As described above, the image information may include information on fluorescence images and/or information on luminescence images. The computing device determines position information against information on the labeled products including the biological specimens or image information including the fluorescent or luminescent material.

The image information can be automatically captured and provided by a specially designed software. Alternatively, the image information may be provided by real-time observation with a microscope. Here, the microscope lens has a magnification sufficient to observe desired biological specimens needing to be distinguished or labeled products including these biological specimens. The observation can be done in the dark field as well as in the bright field with a light source. The labeled products provided through the image information or real-time observation with a microscope can be repeatedly imaged as many as necessary.

Then, the computing device determines whether the determined information corresponding to the type information matches information on the biological specimens (see 220 of Fig. 2). When the information on the types of the labeled products does not match the information on the biological specimens, the computing device does not select the corresponding biological specimens ("NO" in 220 of Fig. 2).

When the determined information corresponding to the type information matches the information on the biological specimens ("YES" in 220 of Fig. 2), the computing device evaluates the information on the morphologies of adherends included in the image information (230 of Fig. 2). The morphology information may be determined from the shape of the fluorescent or luminescent material or from the shapes of the biological specimens.

Then, when the morphology information included in the image information is identical or close to the regular morphology, the computing device can select the corresponding biological specimen ("YES" in 230 of Fig. 2). The position information is inputted as updated information to the server of the computing device in the order approaching the regular morphology in the morphology information (240 in Fig. 2).

A laser can be applied to the corresponding position to isolate the target in the order of the inputted position information (250 of Fig. 2), and as a result, the target biological specimen can be provided with high purity and efficiency. The energy applied to the corresponding position may be a pulse laser but is not limited thereto. The wavelength band of the laser can be freely selected in the range of 10 nm to 100000 nm and the duration of the laser may be 1 as to 1 ms or 1 fs to 100 ns. This series of processes may be implemented in an automated manner.

The selection of the specific biological specimen from the sample including the labeled products can be implemented using the following selection system. For example, the selection system may include: a database storing information on the coordinates of the labeled products, information on signals (including fluorescence signals) of the labeled products, information on observed images of the labeled products, and information on constituent materials of the labeled products; and a server accessing the information stored in the database to use information on constituent materials of the labeled products so that the biological specimens can be screened based on information on the types of adherends to the labeled products and information on the adhesion morphologies of the adherends provided from the information on coordinates of the labeled products, the information on signals of the labeled products, and the information on observed images of the labeled products.

When the information on adherends does not match the information on biological specimens, the server does not select the biological specimens.

When the information on adherends matches the information on biological specimens, the server evaluates the morphology information included in the information on images of the labeled products.

When the morphology information included in the information on images of the labeled products is close to the regular morphology, the server may select the biological specimens.

Specifically, the isolation processing unit 730 may be implemented, for example, in the form of a laser platform. Fig. 8 is an exemplary diagram of the laser platform. Referring to Fig. 8, the laser platform may include a thermo-hygrostat adapted to protect the biological specimens from damage, a support adapted to immobilize the biological specimens onto a solid, and a laser generator. The biological specimens may be provided from a liquid sample. In this case, the thermo-hygrostat may include a water feeder to replenish water evaporated from the liquid sample including the biological specimens and a humidity controller to measure the temperature or humidity of a region between the solid and the water feeder.

The laser generator of the laser platform may use a pulse laser. Pulse laser ablation or radiation pressure injection may occur by the incident pulse laser. In this case, the direction of propagation of the laser wavelength is substantially the same as the moving direction of the target, making it easier to retrieve the target.

The pulse laser may have a wavelength of 10 to 10,000 nm, preferably 20 to 5,000 nm, more preferably 100 to 2,000 nm. An electromagnetic field in the wavelength range defined above, including the visible range, can transfer sufficient energy without any significant influence on optical elements. Most commercial pulse lasers operate in the above range and are thus easy to realize the system. Also when the substrate uses a sacrificial layer, the technology described herein can be carried out without any substantial change of the system.

The pulse laser may have a pulse duration in the range of 1 as to 1 ms, preferably 1 fs to 100 ns. When pulse laser ablation occurs by the pulse laser having a pulse duration in the range defined above, the propagation paths of the separated substrate and the target are made more constant, making it easy to retrieve the target. The separation of the desired target specimen from the substrate includes transferring the desired selected specimen or the unselected specimens to a reservoir. The transfer to the reservoir is necessary to store the separated target specimen and use it when reactions with other reactants are required. The reservoir may include a container designed to cause or observe physical or chemical reactions. The reservoir may include a container designed to store the biochemical molecules. The reservoir has a volume of 1 aL to 1 L, preferably 1 fL to 10 mL, more preferably 10 pL to 500 µL, which corresponds to a reaction volume in which post-processing of the target specimen can be most easily performed. The reservoir may be an array of microwells, each of which has a volume of 1 pL to 1 µL. This array structure can reduce the reaction volume for chemical reactions of the separated molecular clones and the nucleic acids to minimize the waste of reagents. In addition, improved reaction rates and efficiencies in some of the reactions can be expected.

According to the present invention, an object recognition algorithm to analyze images and recognize the shapes, types, and positions of an agent for labeling biological specimens is used in conjunction with an operable arithmetic unit, achieving automation of the extraction process. As a result, the present invention can avoid the need for a separate extraction tool and can improve manual processing that is used in combination or in conjunction with an extraction tool and a system for image observation and storage.

The system for screening and separating biological specimens according to one embodiment of the technology disclosed in the present invention can selectively isolate minimum units of biochemical molecules such as nucleic acids, proteins, and cells. Due to this advantage, the system can be used in a variety of applications. That is, the system can be applied to bioassay and genetic diagnostic systems for selectively isolating desired biological specimens.

The present invention is embodied by the following examples. However, the following experimental data are set forth for illustrative purposes and do not serve to limit the present invention.

### <Example 1>

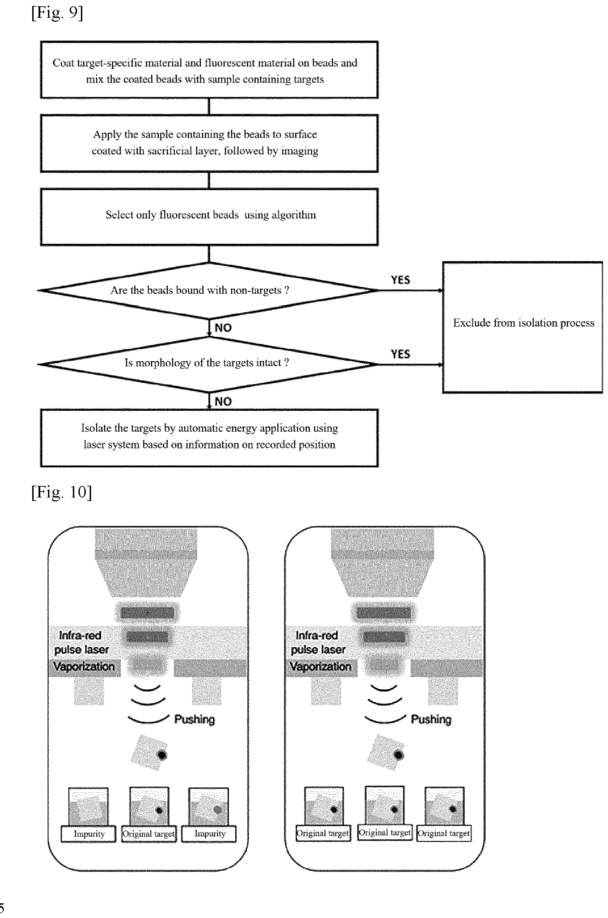

A specific experiment of the present invention was conducted according to the flowchart shown in Fig. 9.

Home-made 10 µm-diameter silica beads and anti-EpCAM antibody (Epithelial Cell Adhesion Molecule, Abcam) were used as specific affinity materials. FITC fluorescent dye as a fluorescent material was coated on the beads, added to a PBS spiked sample containing SKBR3, a breast cancer cell line, followed by labeling. Shaking was performed for sufficient binding to obtain a liquid sample including labeled products.

The liquid sample including labeled products was applied to the surface of a slide glass substrate coated with ITO as a sacrificial layer, and image information was obtained using an optical microscope.

Based on the image information, only the fluorescent beads were selected according to the algorithm shown in Fig. 2. Specifically, if it was determined that the beads were unbound to the biological specimens ("NO"), the biological specimens were excluded from the subsequent isolation process. If it was determined that the beads were bound to the biological specimens ("YES"), a determination was made as to whether the morphology of the biological specimens remained intact close to the regular morphology. If it was determined that the morphology of the biological specimens did not remain intact ("NO"), the biological specimens were excluded from the subsequent isolation process. If it was determined that the biological specimens remained intact ("YES"), an experiment was conducted using the laser platform having the construction illustrated in Fig. 8 based on the information on recorded positions under constant temperature and humidity conditions without causing damage to the biological specimens.

### <Comparative Example 1>

The procedure of Example 1 was repeated except that no determination was made as to whether the morphology of the biological specimens remained intact close to the regular morphology.

That is, based on the image information of Example 1, only fluorescent beads were selected according to the algorithm shown in Fig. 2. Specifically, if it was determined that the beads were unbound to the biological specimens ("NO"), the biological specimens were excluded from the subsequent isolation process. If it was determined that the beads were bound to the biological specimens ("YES"), an experiment was conducted using the laser platform having the construction illustrated in Fig. 8 based on the information on recorded positions under constant temperature and humidity conditions without causing damage to the biological specimens.

### <Experimental Example 1>

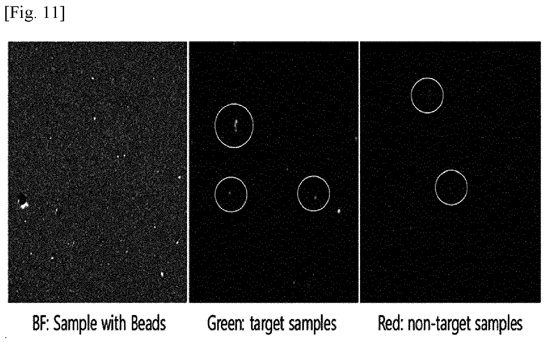

Fig. 10 schematically shows the isolation of the biological specimens with the laser generator in (a) Example 1 and (b) Comparative Example 1 and Fig. 11 shows a high magnification image and fluorescence images of the isolated biological specimens. As shown in (a) of Fig. 10, the biological specimen was isolated with high purity and efficiency and their shape and type were intact. In contrast, some of the isolated biological specimens were not intact in shape, as shown in (b) of Fig. 10. Fig. 11 shows images of the sample with beads (left), the selected green fluorescent molecules (middle), and non-target samples (right).

## Claims

1. A method for screening and separating biological specimens, comprising: adding an agent to a sample comprising biological specimens to label the biological specimens and obtain a sample comprising labeled products; reading the types, shapes, and positions of the labeled products in the sample to select at least one target specimen among the labeled products; and isolating the selected target specimen or the unselected specimens.

2. A method for screening and separating biological specimens, comprising: adding an agent to a sample comprising biological specimens to label the biological specimens and obtain a sample comprising labeled products; immobilizing the sample onto a substrate and reading the types, shapes, and positions of the labeled products to select at least one target specimen among the labeled products; and isolating the selected target specimen or the unselected specimens.

3. The method according to claim 1 or 2, wherein the biological specimens are selected from tissues, cells, nucleic acids, proteins, exosomes, metabolites, and mixtures thereof and the sample is derived from a natural environment or a living organism.

4. The method according to claim 1 or 2, wherein the agent comprises an affinity material for the biological specimens, a location information tracker or a combination thereof.

5. The method according to claim 4, wherein the affinity material for the biological specimens is selected from beads, fluorescent molecules, luminescent molecules, antibodies, aptamers, and combinations thereof.

6. The method according to claim 4, wherein the location information tracker is selected from beads, fluorescent molecules, luminescent molecules, quantum dots, antibodies, aptamers, enzymes, DNAs, RNAs, PNAs, and combinations thereof.

7. The method according to claim 2, wherein the substrate is made of glass, silicon or a polymeric material and comprises a sacrificial layer.

8. The method according to claim 1 or 2, wherein the step of selecting at least one target among the labeled products is carried out by direct visual observation through optical microscopy or electron microscopy or by automated image processing to acquire information on the types, shapes, and positions of the labeled products.

9. The method according to claim 1 or 2, wherein the step of reading the types, shapes, and positions of the labeled products is carried out through a combination of information on observed images, information on signals, comprising fluorescence signals, and information on coordinates.

10. The method according to claim 1 or 2, wherein the step of reading the types, shapes, and positions of the labeled products provides updated information by accumulating information on signals, comprising fluorescence signals, and information on observed images on information on coordinates.

11. The method according to claim 10, wherein the information updating is performed through a selection system comprising: a database storing information on the coordinates of the labeled products, information on signals of the labeled products, information on observed images of the labeled products, and information on constituent materials of the labeled products; and a server accessing the information stored in the database to use information on constituent materials of the labeled products so that the biological specimens are screened based on information on the types of adherends to the labeled products and information on the adhesion morphologies of the adherends provided from the information on coordinates of the labeled products, the information on signals of the labeled products, and the information on observed images of the labeled products.

12. The method according to claim 11, wherein when the information on the type of an adherend provided from information on signals of the labeled products does not match information on the biological specimens as constituent materials of the labeled products, the server does not select the biological specimens.

13. The method according to claim 11, wherein when information on the type of an adherend provided from information on signals of the labeled products matches information on the biological specimens as constituent materials of the labeled products, the server evaluates the information on the adhesion morphology of the adherend.

14. The method according to claim 13, wherein when information on the adhesion morphology of the adherend is the regular morphology, the server selects the corresponding biological specimen.

15. The method according to claim 14, wherein the information on the selected target specimen is updated on information on observed images of the labeled products and the corresponding updated information is included in the database.

16. The method according to claim 15, wherein the target specimen is retrieved in an automated manner by applying one or more isolation means selected from the group consisting of ultrasonic wave, radiation pressure, laser, pulling, and absorption to the target specimen based on the updated information.

17. A system for screening and separating biological specimens, comprising: a stage mounted with a substrate on which a sample comprising labeled products is arranged; an image processing unit adapted to observe the labeled products and select at least one target specimen; and an isolation processing unit adapted to isolate the target selected in the image processing unit.

18. The system according to claim 17, wherein the image processing unit is implemented by a selection system comprising: a database storing information on the coordinates of the labeled products, information on signals of the labeled products, information on observed images of the labeled products, and information on constituent materials of the labeled products; and a server accessing the information stored in the database to use information on constituent materials of the labeled products so that the biological specimens are screened based on information on the types of adherends to the labeled products and information on the adhesion morphologies of the adherends provided from the information on coordinates of the labeled products, the information on signals of the labeled products, and the information on observed images of the labeled products.

19. The system according to claim 17, wherein the isolation processing unit is implemented in the form of a laser platform which comprises a thermo-hygrostat adapted to protect the biological specimens from damage, a support adapted to immobilize the biological specimens onto a solid, and a laser generator, and the thermo-hygrostat comprises a water feeder to replenish water evaporated from a liquid sample comprising the biological specimens and a humidity controller to measure the temperature or humidity of a region between the solid and the water feeder.

20. The system according to claim 19, wherein the laser generator generates a laser having a wavelength band of 10 nm to 100000 nm and a duration of 1 as to 1 ms.
